# EUROPEAN PATENT APPLICATION

(11) **EP 3 892 195 A2**
(43) Date of publication of application: **13.10.2021**
(21) Application number: 19893501.7
(22) Date of filing: 05.12.2019
(51) Int. Cl.: A61B 5/11

(54) **PRESSURE-MEASURING FOOT BOARD, AND EXERCISE APPARATUS COMPRISING SAME FOR CORRECTING STATE OF IMBALANCE OF BODY**

(30) Priority: 06.12.2018 KR 20180156399
(71) Applicant: Lee, Jung Woo, Ulsan 44923 (KR); Moon, Yang Gyu, Ulsan 44923 (KR)
(72) Inventor: KIM, Kwan Myung, Ulju-gun Ulsan 44919 (KR); LEE, Hui Sung, Ulju-gun Ulsan 44919 (KR); PARK, Sang Jin, Ulju-gun Ulsan 44919 (KR); LEE, Sung Ho, Ulju-gun Ulsan 44919 (KR); JEONG, Won Do, Ulju-gun Ulsan 44919 (KR); KIM, Byoung Hern, Ulju-gun Ulsan 44919 (KR)
(74) Representative: Zacco Sweden AB
(86) International application number: PCT/KR2019/017126
(87) International publication number: WO 2020/116970

(57) **Abstract**

The present invention relates to a pressure-measuring foot board having a sturdy structure which may be used in an exercise apparatus capable of precisely measuring and correcting the state of imbalance of the body of a user, and to an exercise apparatus for correcting the state of imbalance of the body comprising the pressure-measuring foot board. The pressure-measuring foot board according to the present invention may firmly fix a pressure-measuring element for measuring the state of imbalance of the body of a user while also preventing or minimizing damage to the elements of the foot board, including the pressure-measuring element, when the foot board is being tilted for balancing exercises. Moreover, an exercise apparatus for correcting the state of imbalance of the body may be provided which can, by means of the pressure-measuring foot board, precisely measure the state of imbalance of the body, and, on the basis thereof, provide corrective exercises that are suitable for the amount of imbalance of each individual's body.

## Description

### Field

The present disclosure relates to a pressure-measuring foot board of a rigid structure that may be applied to an exercise apparatus capable of accurately measuring and correcting a user's body imbalance, and an exercise apparatus including the same for correcting the body imbalance.

### Description of Related Art

Body imbalance is a phenomenon in which body joints are bent or twisted in one direction. It is difficult to remove the body imbalance with a simple diet or exercise. Body imbalance occurs due to various causes, especially due to improper habits or postures. Due to repeated wrong habits, such as crossing your legs, pelvis will gradually twist, which may stimulate nerves and strain muscles. Various exercise methods and exercise apparatuses are being developed to correct this body imbalance. There have been developed a number of devices used for exercise and stretching that may relax the leg muscles that support the weight.

In this regard, Korean Patent No. 10-1390213 discloses a combined exercise and game device provided with a pressure sensor on a bottom face thereof and a foot board that measures a movement of a user's foot via the pressure sensor.

However, this approach simply discloses whether or not a pressure-sensitive sensor is provided, and does not suggest a solution for preventing damage to the foot board and the pressure-sensitive member that may occur during actual operation.

In one example, Korean Patent No. 10-1806163 discloses a lower body stretching exercise apparatus capable of preventing safety accidents while releasing fatigue of legs including ankles and thighs via stretching.

However, this approach allows the leg to be stretched by simply changing an angle of the foot board, and does not suggest an exercise type for correcting an individual's body imbalance state.

### Disclosure

### Technical Purpose

Therefore, in order to solve the above problems, the present disclosure has a purpose to provide a pressure-measuring foot board which may securely fix a pressure-measuring member for measuring the user's body imbalance thereto while preventing or minimizing damage to members thereof including the pressure-measuring member when tilting the foot board for imbalance corrective exercise.

Further, the present disclosure has another purpose to provide an exercise apparatus for correcting the body imbalance that may accurately measure the body imbalance state with the pressure-measuring foot board and provide an imbalance corrective exercise suitable for each individual's body imbalance level based on this measurement.

### Technical Solution

A first aspect of the present disclosure provides a pressure-measuring foot board comprising: a lower plate having a plurality of pressure-measuring members; an upper plate disposed above the lower plate and spaced apart from a top face of the lower plate, wherein the upper plate has each opening defined therein at a position corresponding to a position of each of the pressure-measuring members; and a cover plate positioned on the upper plate, wherein the cover plate has each pressing member passing through each opening and thus pressing a top face of each pressure-measuring member.

A second aspect of the present disclosure provides an exercise apparatus for correcting a body imbalance, the apparatus comprising: the pressure-measuring foot board as defined above; a lower frame having a load support member coupled with a pivot bracket of the pressure-measuring foot board; an actuator installed on the lower frame and having a cylinder performing a reciprocating motion; a connection plate having one end pivotably connected to a distal end of the cylinder, and the opposite end connected to a driving bracket of the pressure-measuring foot board; and a controller configured to analyze a body imbalance value based on a measurement signal received from the pressure-measuring member, and to control the actuator based on the analysis result to tilt the pressure-measuring foot board.

### Technical Effects

The present disclosure may realize the pressure-measuring foot board which may securely fix the pressure-measuring member for measuring the user's body imbalance thereto while preventing or minimizing damage to members thereof including the pressure-measuring member when tilting the foot board for imbalance corrective exercise.

Further, the present disclosure may realize the exercise apparatus for correcting the body imbalance that may accurately measure the body imbalance state and provide the imbalance corrective exercise suitable for each individual's body imbalance level based on this measurement.

### Brief Descriptions of Drawings

FIG. 1 shows a perspective view of a pressure-measuring foot board according to one embodiment of the present disclosure.
FIG. 2 schematically shows a side cross-sectional view of a pressure-measuring foot board according to one embodiment of the present disclosure.
FIG. 3 is a perspective view showing a pressure-measuring foot board according to one embodiment of the present disclosure while a cover plate is removed therefrom.
FIG. 4 schematically shows an exercise apparatus for correcting a body imbalance according to one embodiment of the present disclosure.
FIG. 5 schematically shows a method for operating the exercise apparatus for correcting the body imbalance according to one embodiment of the present disclosure.
FIG. 6 shows one embodiment of a system to which the exercise apparatus for correcting the body imbalance according to the present disclosure is applied.

### Detailed Descriptions

Examples of various embodiments are illustrated and described further below. It will be understood that the description herein is not intended to limit the claims to the specific embodiments described. On the contrary, it is intended to cover alternatives, modifications, and equivalents as may be included within the spirit and scope of the present disclosure as defined by the appended claims.

It will be understood that, although the terms "first", "second", "third", and so on may be used herein to describe various elements, components, regions, layers and/or sections, these elements, components, regions, layers and/or sections should not be limited by these terms. These terms are used to distinguish one element, component, region, layer or section from another element, component, region, layer or section. Thus, a first element, component, region, layer or section described below could be termed a second element, component, region, layer or section, without departing from the spirit and scope of the present disclosure.

The terminology used herein is for the purpose of describing particular embodiments only and is not intended to be limiting of the present disclosure. As used herein, the singular forms "a" and "an" are intended to include the plural forms as well, unless the context clearly indicates otherwise. It will be further understood that the terms "comprises", "comprising", "includes", and "including" when used in this specification, specify the presence of the stated features, integers, operations, elements, and/or components, but do not preclude the presence or addition of one or more other features, integers, operations, elements, components, and/or portions thereof. As used herein, the term "and/or" includes any and all combinations of one or more of the associated listed items. Expression such as "at least one of' when preceding a list of elements may modify the entire list of elements and may not modify the individual elements of the list.

Prior to the detailed description of the drawings, it is intended to clarify that components in the present specification are merely defined based on a main function that each component is responsible for. Thus, two or more components may be combined into one component, or one component may be divided into two or more sub-components for sub-functions.

Each component to be described below may additionally perform some or all of functions that other components are responsible for, in addition to its own main function. Some of the main functions that each component is responsible for may be dedicated to other components. Therefore, whether or not each component as described in the present specification exists should be functionally interpreted.

A first aspect of the present disclosure provides a pressure-measuring foot board comprising: a lower plate having a plurality of pressure-measuring members; an upper plate disposed above the lower plate and spaced apart from a top face of the lower plate, wherein the upper plate has each opening defined therein at a position corresponding to a position of each of the pressure-measuring members; and a cover plate positioned on the upper plate, wherein the cover plate has each pressing member passing through each opening and thus pressing a top face of each pressure-measuring member.

In one implementation of the pressure-measuring foot board, the lower plate has a pivot bracket coupled to a bottom face thereof, wherein the pivotable bracket acts as a pivoting axis when the pressure-measuring foot board is tilted.

In one implementation of the pressure-measuring foot board, each pressure-measuring member is fixed to the lower plate via a pressure-measuring member bracket and a fastener.

In one implementation of the pressure-measuring foot board, the plurality of pressure-measuring members respectively contact front and rear ends of feet of a user.

A second aspect of the present disclosure provides an exercise apparatus for correcting a body imbalance, the apparatus comprising: the pressure-measuring foot board as defined above; a lower frame having a load support member coupled with a pivot bracket of the pressure-measuring foot board; an actuator installed on the lower frame and having a cylinder performing a reciprocating motion; a connection plate having one end pivotably connected to a distal end of the cylinder, and the opposite end connected to a driving bracket of the pressure-measuring foot board; and a controller configured to analyze a body imbalance value based on a measurement signal received from the pressure-measuring member, and to control the actuator based on the analysis result to tilt the pressure-measuring foot board.

In one implementation of the apparatus, the controller of the exercise apparatus for correcting the body imbalance is configured to: use the pressure-measuring members to detect a center of gravity of the body of the user in front, rear, left, and right directions based on both feet of the user; compare the detected center of gravity with a reference value stored in the controller; and measure the body imbalance based on the comparing result.

In one implementation of the apparatus, the apparatus further comprises a foot board support member installed on the lower frame to support and maintain the pressure-measuring foot board in a leveled state when measuring the user's body imbalance.

Hereinafter, the present disclosure will be described in more detail with reference to the attached drawing to help understand the present disclosure. However, the following embodiment are provided for easier understanding of the present disclosure, and the contents of the present disclosure are not limited to the following embodiments.

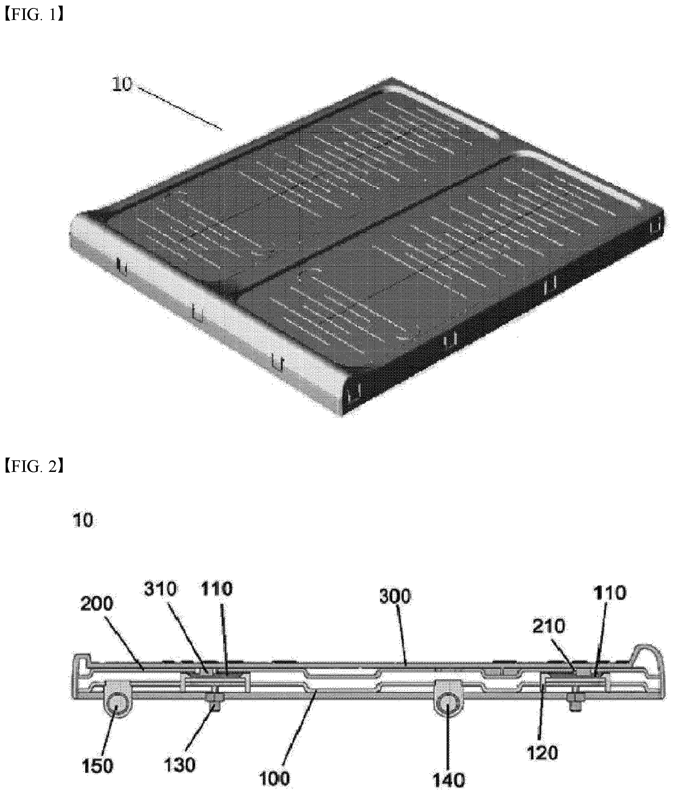

FIGS. 1 to 3 show the pressure-measuring foot board according to one embodiment of the present disclosure. When described with reference to FIGS. 1 to 3, a pressure-measuring foot board 10 according to the present disclosure includes a lower plate 100 having a plurality of pressure-measuring members 110, an upper plate 200 disposed above the lower plate 100 and spaced apart from a top face of the lower plate 100, wherein the upper plate 200 has each opening 210 defined therein at a position corresponding to a position of each of the pressure-measuring members 110, and a cover plate 300 positioned on the upper plate 200, wherein the cover plate 300 has each pressing member 310 passing through each opening 210 and thus pressing a top face of each pressure-measuring member 110.

The pressure-measuring member 110 may include at least two pressure-measuring members 110 which may be respectively in contact with a front end and a rear end of a foot of a user while the user is standing on the pressure-measuring foot board 10 in order to accurately measure the user's body imbalance. The pressure-measuring member 110 may be embodied as a load cell or a pressure measuring pad. However, the present disclosure is not limited thereto. The pressure-measuring member 110 may be fixed to the lower plate 100 using a pressure-measuring member bracket 120 and a fastener 130. The pressure-measuring member 110 may be fixed to the lower plate 100 via the pressure-measuring member bracket 120 without being separated from the lower plate 100 even when the pressure-measuring foot board 10 is tilted.

The upper plate 200 prevents the cover plate 300 from slipping or twisting due to a pressure applied from the user when the pressure-measuring foot board 10 is tilted, and prevent the pressure-measuring member 110 from being damaged by the applied pressure. The upper plate 200 may be disposed above the lower plate and may be spaced apart from a top face of the lower plate 100 so that the pressure-measuring member 110 and the pressure-measuring member bracket 120 may be installed between the lower plate 100 and the upper plate 200. The upper plate 200 may be fixed to the lower plate 100 via the fastener 130 while being spaced from the lower plate 100. The upper plate 200 may have each opening 210 defined therein at a position corresponding to that of each of the pressure-measuring members 110. Thus, each pressing member 310 provided on the cover plate 300 may be received in each opening 210 and then may press a top face of each pressure-measuring member 110 to measure the user's body imbalance. In order to prevent the cover plate 300 from twisting when the pressure-measuring foot board is tilted, a width of the opening may be sized such that the pressing member 310 is tightly fitted into the opening.

The cover plate 300 may be formed in a form surrounding a side edge of each of the lower plate 100 and the upper plate 200 to prevent twisting or separation of the foot board while the foot board is tilted. The cover plate 300 may be formed in a shape such as a rectangle or an oval, or in a shape of a human foot or other various shapes. Further, in order to prevent the user from slipping on and along the cover plate 300, an embossed or engraved pattern may be formed in a top face thereof.

In this way, the pressure-measuring foot board 10 according to the present disclosure includes the lower plate 100 provided with the plurality of pressure-measuring members 110 and the upper plate 200 provided with the plurality of openings 210, that is, has a double structure. Further, the separate cover plate 300 having the plurality of the pressing members 310 may be disposed on the upper plate 200. Thus, the pressure-measuring foot board 10 may accurately measure the user's body imbalance and may firmly fix the pressure-measuring member 110 therein. When the pressure-measuring foot board 910 is tilted for the imbalance corrective exercise, the pressure-measuring foot board 910 may prevent or minimize the separation, twisting, or damage of each of the members of the pressure-measuring foot board 10 due to the pressure applied from the user in the inclined direction. In particular, the pressure-measuring foot board 910 may prevent the damage to the pressure measuring member 110.

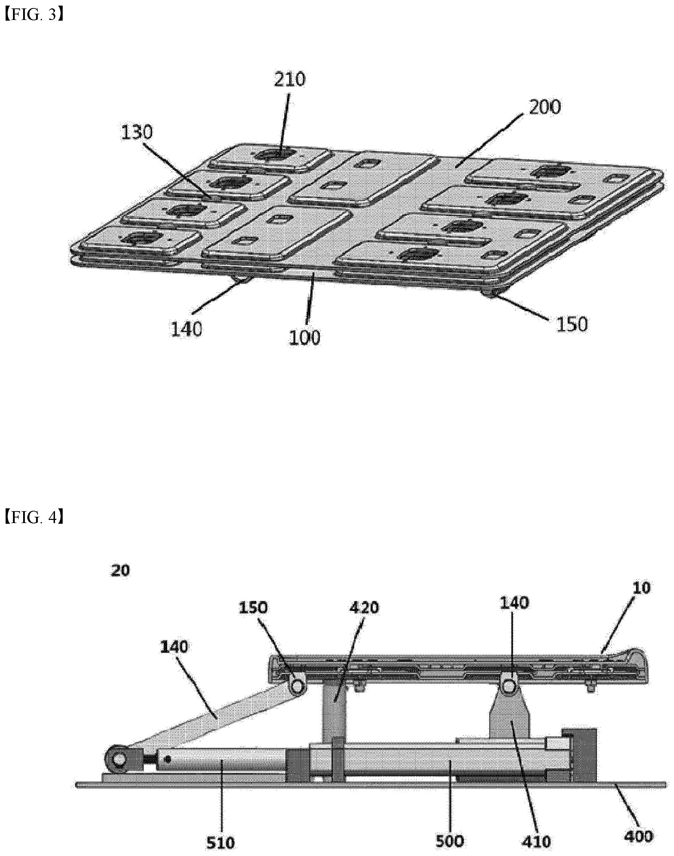

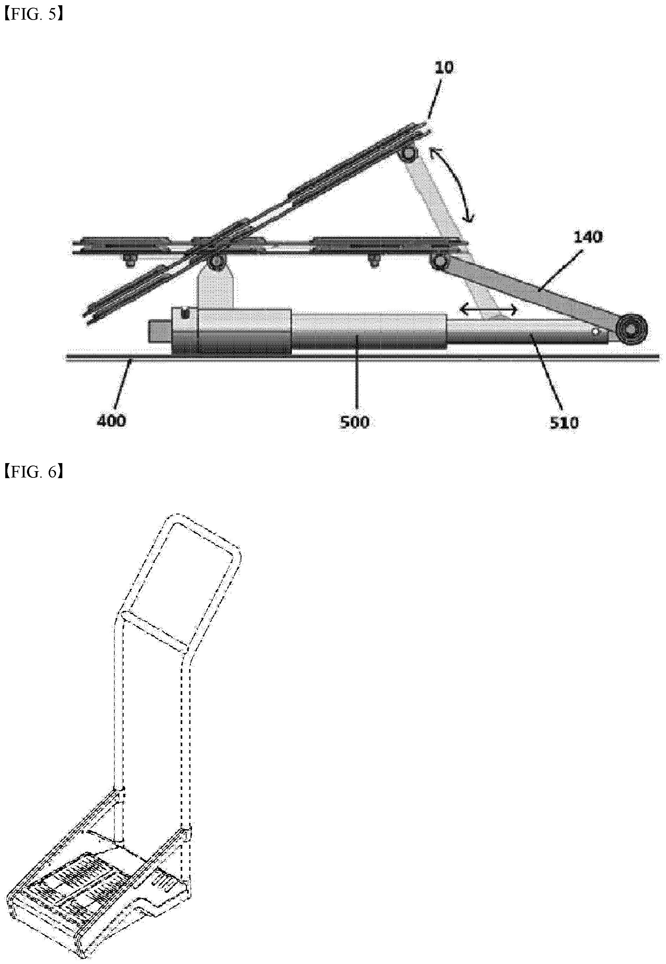

FIG. 4 schematically shows an exercise apparatus for correcting a body imbalance according to one embodiment of the present disclosure. FIG. 5 schematically shows a method for operating the exercise apparatus for correcting the body imbalance according to one embodiment of the present disclosure. FIG. 6 shows one embodiment of a system to which the exercise apparatus for correcting the body imbalance according to the present disclosure is applied. When described with reference to FIGS. 4 to 6, an exercise apparatus 20 for correcting a body imbalance according to the present disclosure may include the pressure-measuring foot board 10 as described above, a lower frame 400 having a load support member 410 coupled with a pivot bracket 140 of the pressure-measuring foot board 10, an actuator 500 installed on the lower frame 400 and having a cylinder 510 performing a reciprocating motion, a connection plate 600 having one end pivotably connected to a distal end of the cylinder 510, and the opposite end connected to a driving bracket 150 of the pressure-measuring foot board 10, and a controller 700 configured to analyze a body imbalance value based on a measurement signal received from the pressure-measuring member 110, and to control the actuator 500 to tilt the pressure-measuring foot board 10.

The actuator 500 provides power to tilt the pressure-measuring foot board 10 and is fixedly installed on the lower frame 400, and may be fixed thereto using a separate fixing bracket. The actuator 500 may extend horizontally and in parallel to a bottom face of the pressure-measuring foot board 10 to minimize a volume of the exercise apparatus 20 for correcting the body imbalance. The actuator 500 has a cylinder 510 capable of reciprocating motion.

The connection plate 600 has one end pivotally connected to the distal end of the cylinder 510 and the opposite end connected to the driving bracket 150 of the pressure-measuring foot board 10. Due to this connection structure, the connection plate 600 may receive a driving force from the piston 510 which performs a horizontal linear movement under the operation of the actuator 500, and convert the linear moving force into a pivoting force to tilt the pressure-measuring foot board 10.

In one example, the exercise apparatus 20 for correcting the body imbalance according to the present disclosure may guide the user to perform a milking action when the user steps on the pressure-measuring foot board 10. In other words, while the exercise apparatus 20 maintains the pressure-measuring foot board 10 at a specific angle and direction, the exercise apparatus 20 reciprocates the board 10 in the vertical direction so that the milking action may be performed on a specific body part such as the ankle or calf of the user. In another example, the exercise apparatus 20 may change the direction, the angle or the moving distance of the foot board 10 to maximize the exercise effect during the reciprocating motion.

A body imbalance correcting method using the exercise apparatus 20 for correcting the body imbalance according to the present disclosure may be implemented as follows. The amount of the user's initial body imbalance is measured using the pressure-measuring member 110 provided on the pressure-measuring foot board 10 that the user may step on. In one embodiment, the exercise apparatus 20 for correcting the body imbalance may detect a center of gravity in front, rear, left and right directions of the user body, based on both feet of the user using the pressure-measuring foot board 10 equipped with the plurality of pressure-measuring members 110 and may measure an amount of the initial body imbalance based on a comparing result between the sensed center of gravity and a previously stored reference value.

In general, the body imbalance may be associated with symptoms such as turtleneck or scoliosis, or a pain of a specific part of the body. Alternatively, pain or lesions may occur due to the imbalanced body state. The exercise apparatus 20 for correcting the body imbalance according to the present disclosure may detect the amount of the user's initial body imbalance.

In one example, the exercise apparatus 20 for correcting the body imbalance according to the present disclosure may use a communication module to transmit the measured value about the initial body imbalance. In this connection, the communication module may be configured for receiving specific information from an external terminal such as a user's smart phone, or a terminal associated with the exercise apparatus 20 for correcting the body imbalance or for transmitting a measurement value about the body balance. For example, the communication module capable of transmitting and receiving data over a Bluetooth or Wi-Fi network may be used.

Further, the exercise apparatus 20 for correcting the body imbalance according to the present disclosure may transmit a trigger signal to trigger the measurement to the terminal so that the amount of the user's initial body imbalance may be measured in an accurate environment when measuring the amount of the user's initial body imbalance. That is, the terminal may allow start of the operation for body imbalance correcting of the user upon receiving the trigger signal transmitted from the exercise apparatus 20 for correcting the body imbalance, and may provide related guide information. The terminal and the exercise apparatus 20 for correcting the body imbalance are wirelessly connected to each other. Thus, when the apparatus 20 provides information on the posture or position for measurement through the screen of the terminal, the user may measure the initial imbalance of the body at the correct posture at the correct point on the foot board.

Further, the exercise apparatus 20 for correcting the body imbalance according to the present disclosure may be connected to a separate exercise information server. The exercise information server may include a processor capable of calculating a setting value, and thus may calculate a setting value for the operation of the exercise apparatus for correcting the body imbalance, based on the measured initial body imbalance information. Based on the measurement information and the calculated setting value, the server may calculate at least one of the direction, angle, and number of milking actions of the pressure-measuring foot board 10 as a setting value. For example, when the user's body imbalance is inclined in a specific direction, a setting value for distributing the center of gravity may be calculated by moving the angle of the pressure-measuring foot board 10 in a corresponding direction or tilting the board 10 in a corresponding direction. The exercise apparatus 20 for correcting the body imbalance may set or operate the pressure-measuring foot board 10 upon receiving the calculated setting value.

The pressure-measuring foot board and the exercise apparatus for correcting the body imbalance using the same according to one embodiment of the disclosed technology have been described with reference to the embodiments shown in the drawings for better understanding. However, those are only exemplary. Those of ordinary skill in the art will understand that various modifications and equivalent other embodiments are possible therefrom. Therefore, the true technical protection scope of the present disclosure should be determined by the appended claims.

## Claims

1. A pressure-measuring foot board comprising:
a lower plate having a plurality of pressure-measuring members;
an upper plate disposed above the lower plate and spaced apart from a top face of the lower plate, wherein the upper plate has each opening defined therein at a position corresponding to a position of each of the pressure-measuring members; and
a cover plate positioned on the upper plate, wherein the cover plate has each pressing member passing through each opening and thus pressing a top face of each pressure-measuring member.

2. The pressure-measuring foot board of claim 1, wherein the lower plate has a pivot bracket coupled to a bottom face thereof, wherein the pivotable bracket acts as a pivoting axis when the pressure-measuring foot board is tilted.

3. The pressure-measuring foot board of claim 2, wherein each pressure-measuring member is fixed to the lower plate via a pressure-measuring member bracket and a fastener.

4. The pressure-measuring foot board of claim 2, wherein the plurality of pressure-measuring members respectively contact front and rear ends of feet of a user.

5. An exercise apparatus for correcting a body imbalance, the apparatus comprising:
the pressure-measuring foot board according to one of claims 2 to 4;
a lower frame having a load support member coupled with a pivot bracket of the pressure-measuring foot board;
an actuator installed on the lower frame and having a cylinder performing a reciprocating motion;
a connection plate having one end pivotably connected to a distal end of the cylinder, and the opposite end connected to a driving bracket of the pressure-measuring foot board; and
a controller configured to analyze a body imbalance value based on a measurement signal received from the pressure-measuring member, and to control the actuator based on the analysis result to tilt the pressure-measuring foot board.

6. The apparatus of claim 5, wherein the controller of the exercise apparatus for correcting the body imbalance is configured to:
use the pressure-measuring members to detect a center of gravity of the body of the user in front, rear, left, and right directions based on both feet of the user;
compare the detected center of gravity with a reference value stored in the controller; and
measure the body imbalance based on the comparing result.

7. The apparatus of claim 5, wherein the apparatus further comprises a foot board support member installed on the lower frame to support and maintain the pressure-measuring foot board in a leveled state when measuring the user's body imbalance.
